(19) Europäisches Patentamt
European Patent Office
Office européen des brevets

(11) EP 2 413 719 B1

(12) # FASCICULE DE BREVET EUROPEEN

(45) Date de publication et mention de la délivrance du brevet:
**08.07.2015 Bulletin 2015/28**

(21) Numéro de dépôt: **10714072.5**

(22) Date de dépôt: **30.03.2010**

(51) Int Cl.:
***A61K 9/51*** *(2006.01)*

(86) Numéro de dépôt international:
**PCT/IB2010/051377**

(87) Numéro de publication internationale:
**WO 2010/113111 (07.10.2010 Gazette 2010/40)**

(54) **PROCEDE DE PREPARATION DE CAPSULES LIPIDIQUES FONCTIONNALISEES**

VERFAHREN ZUR HERSTELLUNG VON FUNKTIONALISIERTEN LIPIDKAPSELN

METHOD FOR PREPARING FUNCTIONALIZED LIPID CAPSULES

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **31.03.2009 FR 0952048**

(43) Date de publication de la demande:
**08.02.2012 Bulletin 2012/06**

(73) Titulaires:
- **UNIVERSITE D’ANGERS**
**49000 Angers (FR)**
- **INSTITUT NATIONAL DE LA SANTE ET DE LA RECHERCHE MEDICALE (INSERM)**
**75013 Paris (FR)**

(72) Inventeurs:
- **BENOIT, Jean-Pierre**
**F-49100 Angers (FR)**
- **PERRIER, Thomas**
**F-49100 Angers (FR)**

(74) Mandataire: **Le Coupanec, Pascale A.M.P.**
**Cabinet Nony**
**3, rue de Penthièvre**
**75008 Paris (FR)**

(56) Documents cités:
**WO-A-01/64328**
**WO-A-2007/106683**
**FR-A- 2 677 272**
**US-A- 5 635 609**
**US-A1- 2007 140 965**
**US-A1- 2007 184 076**
**US-A1- 2008 160 094**
**US-A1- 2008 253 960**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention vise à proposer de nouveaux systèmes vésiculaires et notamment de type capsules à enveloppe lipidique solide et coeur lipidique liquide.

Des systèmes de type nanocapsules ou nanogouttelettes dont la taille varie de 50 à 500 nanomètres et formés d'un coeur liquide ou semi-solide, enveloppé d'une membrane externe, sont déjà connus.

Par exemple, le brevet US 5,961,970 propose à titre de véhicule d'actifs, des émussions huile-dans-eau à échelle submicronique, c'est-à-dire des mini-émulsions dont les gouttelettes possèdent un noyau hydrophobe de nature lipidique et sont stabilisées en surface par des tensioactifs amphiphiles et/ou non ioniques à l'image des tensioactifs de type phospholipides. Le brevet US 5,576,016 décrit des macroémulsion's dont les gouttelettes sont formées d'un noyau lipidique solide et qui sont stabilisées par une enveloppe phospholipidique. EP 1 265 698, WO 09/001019 et FR 2 916 974, quant à eux, décrivent des nanocapsules à coeur liquide et écorce solide de nature lipidique, pouvant être obtenues à partir d'une microémulsion préparée par la technique d'inversion de phase par effet thermique (évulsion PIT).

US2008 / 253960 concerne des plateformes lipoprotéiques comprenant un coeur lipidique liquide et une écorce solide constituée de phospholipides, de cholestérols non estérifiés, et d'une protéine, en particulier une apoprotéine ApoB-100. La réaction de liaison entre l'acide folique et l'ApoB-100 relève d'un couplage peptidique.

WO2007/106683 et FR 2677 272 décrivent des particules lipoprotéiques comprenant un coeur non liquide et une écorce lipidique.

Ces systèmes nanoparticulaires sont, pour l'essentiel, dédiés à l'encapsulation d'un actif qui, en fonction de sa nature, hydrophile, hydrophobe ou hydrodispersible, sera présent dans le coeur et/ou dans l'écorce.

Au sens de la présente invention, on entend par « encapsulation d'un actif », son incorporation au niveau du coeur ou de l'écorce d'une nanocapsule, suivant sa nature, hydrophile, hydrophobe ou hydrodispersible.

Toutefois, ces systèmes vésiculaires ne se prêtent pas simultanément au transport d'une grande variété d'actifs et/ou à une maîtrise totale de leur capacité de relargage et/ou de ciblage. En effet, ces systèmes ne permettent l'encapsulation que d'actifs de poids moléculaire relativement bas, en particulier de poids moléculaire inférieur ou égal à environ 5 000 daltens. Par ailleurs, il serait avantageux de pouvoir renforcer la structure capsulaire de ces nanocapsules pour prévenir par exemple tout relargage intempestif de l'actif qu'elles véhiculent ou encore accroître la résistance de leur enveloppe aux milieux biologiques agressifs à l'image, par exemple, des sucs gastriques. Il pourrait être, en outre, particulièrement intéressant de pouvoir facilement les fonctionnaliser en surface en vue, par exemple, de les dédier à une cible spécifique. Ainsi, il pourrait être particulièrement intéressant de mettre à profit des ligands de type galactose pour diriger ces nanocapsules vers le récepteur exprimé par les hépatocytes ou de type peptide arginine-glycine-aspartate RGD pour cibler les intégrines $\alpha V\beta 3$ exprimés par certaines tumeurs fortement vascularisées.

L'invention vise précisément à proposer un mode de fonctionnalisation de ces systèmes vésiculaires permettant de leur conférer les avantages précités.

De manière inattendue, les inventeurs ont constaté que la surface externe de nanocapsules peut se prêter à une telle fonctionnalisation sous réserve que celle-ci soit réalisée dans des conditions spécifiques.

Contre toute attente, la fonctionnalisation en surface de ces nanocapsules n'affecte pas leur stabilité et n'entraîne pas leur désagrégation, comme confirmé dans les exemples ci-après par les mesures en diffusion dynamique de la lumière ou par l'imagerie par microscopie électronique à transmission.

Ainsi, la présente invention vise, selon un premier de ses aspects, une capsule de type coeur lipidique liquide/écorce lipidique solide fonctionnalisée en surface avec au moins un composé contenant au moins une fonction amine, caractérisée en ce que l'architecture coeur/écorce lipidiques est à l'échelle nanométrique et en ce que ledit composé est lié de manière covalente *via* une réaction de transacylation à ladite écorce lipidique solide.

Selon un autre de ces aspects, la présente invention vise un procédé utile pour la fonctionnalisation de nanocapsules à écorce lipidique solide et coeur lipidique liquide, ledit procédé comprenant au moins les étapes consistant à :

i) disposer de nanocapsules à écorce lipidique solide et coeur lipidique liquide,
ii) mettre en contact lesdites nanocapsules avec une solution aqueuse alcaline, de manière à activer leur surface pour une réaction de transacylation,
iii) mettre en présence le milieu ii) avec au moins un composé contenant au moins une fonction amine pour former, par transacylation, lesdites capsules attendues et, le cas échéant,
iv) neutraliser les capsules fonctionnalisées ainsi obtenues, et
v) isoler lesdites capsules fonctionnalisées.

Certes, EP 0 693 963 décrit la préparation de particules dont l'écorce est dite « gélifiée », à partir de polysaccharides estérifiés, présents dans l'écorce de la particule considérée, et aptes à réagir avec une polyamine via une réaction de transacylation.

Toutefois, outre leur nature très différente de celles considérées selon l'invention, les particules de départ

subissant une réaction de transacylation possèdent une taille allant de quelques micromètres à 10 millimètres.

Or, les particules de taille plus réduite, notamment à l'échelle nanométrique, possèdent avantageusement une grande stabilité colloïdale. Les nanoparticules ainsi fonctionnalisées résistent à la floculation ou à l'agrégation et ont une durée de vie plus longue.

Au sens de la présente invention, on entend par « nanocapsules fonctionnalisées », des nanocapsules à écorce lipidique solide, dont la surface possède une structure modifiée par rapport à la surface des nanocapsules à un état non fonctionnalisé, cette structure modifiée étant notamment le résultat d'un greffage en surface d'au moins un ou plusieurs composés portant au moins une fonction amine.

A des fins de simplification, ces composés peuvent également être désignés ci-après sous la dénomination de composés aminés. Un tel composé peut avantageusement posséder au moins deux, au moins trois, voire plus de fonctions amines.

On comprend aisément que l'ancrage de telles molécules confère à la nanocapsule ainsi fonctionnalisée une taille supérieure à sa taille initiale (c'est-à-dire non fonctionnalisée). Ainsi, si les nanocapsules d'origine possèdent une taille moyenne inférieure à 150 nm, de préférence inférieure à 100 nm, de préférence encore inférieure à 50 nm, les capsules obtenues suite à leur fonctionnalisation peuvent posséder, pour leur part, une taille moyenne supérieure à 150 nm, voire à 200 nm. La maîtrise du procédé, et notamment du temps de réaction, permet de contrôler la taille finale des nanocapsules fonctionnalisées, qui avantageusement peut demeurer inférieure à 250 nm.

Le composé aminé fixé à la surface des capsules selon l'invention peut en outre être propice à la fixation d'une ou plusieurs molécules d'intérêt.

Ainsi, au sens de l'invention, le terme « composé aminé » couvre soit un actif en tant que tel, dit encore molécule d'intérêt, c'est-à-dire une entité dotée d'une activité biologique, pharmacologique, cosmétique, phytosanitaire, soit une entité chimique ou biologique apte à conférer une ou plusieurs fonctionnalités complémentaires aux nanocapsules, et notamment apte à renforcer leur structure externe et/ou à permettre un ciblage spécifique du/des actif(s) associé(s).

Avantageusement, le composé aminé et/ou la molécule d'intérêt est de nature protéique, peptidique, nucléique, polymérique ou inorganique, voire organométallique.

Au regard des nanocapsules considérées, un procédé selon l'invention s'avère tout particulièrement avantageux pour :

- renforcer leur structure nanocapsulaire,
- modifier leur cinétique de relargage des principes actifs encapsulés, le cas échéant,
- accroître leur résistance aux milieux biologiques agressifs tels que le tube digestif,
- protéger le coeur huileux contenant un ou des actifs de toute agression extérieure (oxydation, lumière),
- tirer profit de ces nanoparticules pour véhiculer des molécules de haut poids moléculaire ou des polymères hydrophiles aptes à réagir après transacylation tels que des acides nucléiques, les ARNsi, les héparines et dérivés d'héparines, les protéines et polypeptides chargés négativement ou positivement, les macro-poly-anions, et/ou
- permettre la présentation en surface de ces nanoparticules de molécules interagissant avec le système immunitaire.

La présente invention vise également, selon un autre de ses objets, l'utilisation de capsules selon l'invention pour la préparation de compositions.

Ainsi, l'invention concerne l'utilisation d'au moins une capsule selon l'invention pour la préparation d'une composition à intérêt thérapeutique, cosmétique ou nutracetique.

La présente invention vise donc également des compositions, notamment des compositions pharmaceutiques, contenant au moins une capsule selon l'invention en association avec au moins un véhicule physiologiquement acceptable.

**<u>Capsules</u>**

Dans ce qui suit, « LNC » signifie « Lipidic Nanocapsules » ou « nanocapsules lipidiques ».

Comme indiqué précédemment, les capsules selon l'invention correspondent en fait à des nanocapsules à écorce lipidique solide et coeur lipidique liquide fonctionnalisées en surface *via* un procédé de transacylation.

Les capsules ainsi obtenues, possèdent une taille moyenne comprise entre 70 et 250 nm. Bien entendu, des capsules de taille supérieure peuvent être obtenues en faisant varier, de manière adéquate, les paramètres du procédé.

De telles variations sont bien entendu du ressort de l'homme de l'art.

De telles capsules peuvent être analysées par diffusion dynamique de la lumière et diffusion des neutrons aux petits angles.

Comme indiqué précédemment, l'écorce externe des capsules comporte au moins un composé aminé greffé à sa surface de manière covalente *via* une réaction de transacylation.

Selon la présente invention, les termes « lié » et « greffé » sont utilisés indifféremment pour désigner l'établissement d'une liaison covalente entre l'écorce et le composé aminé, cette liaison dérivant d'une réaction de transacylation établie entre les deux entités.

Selon une première variante, ce composé aminé est, en soit, une molécule d'intérêt, telle que décrite précédemment, qu'il est intéressant de véhiculer via une capsule conforme à l'invention.

Comme précisé ci-dessus, cette molécule d'intérêt peut être de nature protéique, peptidique, nucléique ou polymérique.

Il peut notamment s'agir d'une protéine, de préférence choisie parmi les protéines hydrophiles ou traitées de manière à être rendues hydrophiles, c'est-à-dire solubles dans l'eau ou dispersibles dans l'eau, contenant des groupements aminés libres, des polypeptides ou des polymères.

Des exemples de protéines qui peuvent être utilisées dans l'invention et qui remplissent les conditions qui consistent à être hydrophiles ou bien qui peuvent être traitées pour être hydrophiles, sont les albumines comme la sérum albumine, en particulier la sérum albumine humaine, l'ovalbumine, l'alpha-lactalbumine, des globulines, le fibrinogène, la caséine, des protéines végétales telles que les protéines du soja ou du blé, des gluténines qui de préférence auront été dégradées, des scléroprotéines solubilisées, le collagène, l'atélocollagène, la gélatine, les hydrolysats de gélatine, les peptones, l'hémoglobine, des enzymes telles que la catalase, la phosphatase alcaline, des hormones, des immunoglobulines ou des anticorps tels que les anticorps monoclonaux.

Avantageusement, comme exemples de polypeptide, on peut citer le polyacide aspartique, la polyarginine ou encore la polylysine.

A titre d'exemple de polymère, on peut citer les polymères synthétiques, et plus particulièrement le polyéthylène imine (PEI).

A titre d'exemple d'acide nucléique, on peut plus particulièrement citer l'ARNsi.

Selon une seconde variante qui peut, le cas échéant, être inhérente à la première variante, le composé aminé est mis en ouvre pour véhiculer un actif annexe plus particulièrement considéré au regard de sa capacité de ciblage et/ou de marquage. Dans le cas où le composé aminé est plus particulièrement considéré à ces fins, c'est-à-dire d'agent devant assurer la liaison d'une molécule d'intérêt à des nanocapsules, on peut plus particulièrement privilégier le choix de composés aminés choisis parmi les anticorps monoclonaux, les ligands RGD, les sucres aminés et la polylysine.

En ce qui concerne la molécule d'intérêt figurée par l'actif, elle peut être conforme à la définition proposée ci-dessus pour le composé aminé.

Il peut également s'agir, par exemple, d'une substance intéressant le diagnostic portant par exemple des entités fluorescentes, luminescentes ou phosphorescentes, ou encore d'une molécule dotée d'une activité biologique telle qu'une enzyme, une hormone, un anticorps ou l'hémoglobine.

Selon encore une autre variante, le composé aminé présente un intérêt au regard de sa capacité à renforcer la résistance de surface externe nanocapsulaire.

Il peut ainsi s'agir de toute molécule comportant des motifs polyéthylène glycol. Un enrobage de surface à base d'un tel composé est en effet avantageux pour conférer une rémanence vasculaire accrue en raison d'une réduction significative de la capture des nanocapsules par les macrophages hépatiques.

Selon encore un autre mode de réalisation, les capsules selon l'invention sont en outre avantageuses au regard du fait qu'elles peuvent posséder au niveau de leur surface externe un certain nombre de motifs annexes réactifs, tels que par exemple des motifs amines et acides propices à des couplages à des entités annexes. De tels motifs sont avantageusement présents sur le composé aminé ayant servi à fonctionnaliser les nanocapsules. Ainsi, selon encore une autre variante de réalisation, les composés aminés considérés selon l'invention peuvent avoir pour intérêt significatif de conférer aux nanocapsules d'origine une aptitude à s'associer, *via* leurs motifs amines fixés par transacylation sur la surface de ces nanocapsules, avec d'autre(s) molécule(s) dotée(s), elle(s), d'intérêt en terme d'effet thérapeutique, de marquage, de ciblage et/ou de protection d'une éventuelle dégradation engendrée par le milieu environnant.

Les molécules d'intérêt, susceptibles d'être liées de façon non covalente avec un composé aminé, lui-même greffé en surface de l'écorce lipidique de la capsule, peuvent être soit un actif, en particulier un actif hydrophile, plus particulièrement chargé négativement, soit une molécule dédiée à apporter une fonctionnalité complémentaire à la nanocapsule, notamment en terme de ciblage/marquage ou à des fins de renforcer la résistance et la stabilité de la nanocapsule.

L'actif peut être un composé à intérêt thérapeutique, en particulier pharmaceutiquement actif, cosmétiquement actif ou actif dans un domaine phytosanitaire ou alimentaire ou nutracetique.

Selon un mode réalisation préféré, cet actif est un principe pharmaceutiquement actif, tel que défini précédemment.

Les nanocapsules de l'invention conviennent plus particulièrement pour l'administration des principes actifs suivants : les anti-infectieux parmi lesquels les antimycosiques ; les antibiotiques ; les anticancéreux ; les immunosuppresseurs ; les principes actifs destinés au Système Nerveux Central qui doivent passer la barrière hémato-encéphalique, tels que les antiparkinsoniens, les antalgiques et plus généralement les principes actifs pour traiter les maladies neurodégénératives.

Avantageusement, un tel actif peut être de nature protéique, polypeptidique, peptidique, mais peut aussi être un acide nucléique tel qu'un plasmide d'ADN ou un ARN d'interférence (ou ARNsi), ou un oligonucléotide antisens ou un aptamère. Le cas échéant, les capsules fonctionnalisées par un actif, lié directement ou via un composé aminé annexe à l'écorce lipidique, peuvent subir un traitement supplémentaire visant à fonctionnaliser leur surface externe avec un composé annexe, tel que par exemple une polylysine, afin de renforcer la protection de l'actif fixé.
Les capsules ainsi formées peuvent être assimilées à un système multicouches.
Une telle variante de réalisation est illustrée en exemple 9.
Comme indiqué précédemment, les capsules selon l'invention comprennent une écorce lipidique solide.

Ecorce lipidique solide

Cette écorce lipidique est solide et comprend au moins un composé apte à réagir avec un composé aminé via une réaction de transacylation. Il s'agit généralement d'un composé comprenant au moins une fonction ester.
Ce composé porteur de la fonction ester peut être un composé participant parallèlement à la formation et la constitution de l'écorce lipidique solide, tel que, par exemple, un tensioactif lipophile, voire un composé annexe, introduit au niveau de l'écorce lipidique uniquement à des fins d'établissement de réactions de transacylation.
Ainsi, l'écorce lipidique solide comprend au moins un tensioactif liposoluble ou lipophile.
Avantageusement, le tensioactif lipophile est solide à température ambiante.
Le tensioactif lipophile est plus particulièrement à base de phospholipides qui sont avantageux au regard de leur caractère biocompatible.
Parmi les phospholipides, les phosphatidylcholines (lécithines) sont tout particulièrement intéressantes.
D'autres phospholipides peuvent être le phosphatidylglycérol, le phosphatidylinositol, la phosphatidylsérine, l'acide phosphatidique et la phosphatidyléthanolamine.
Les dérivés phospholipides peuvent être isolés à partir de sources naturelles ou préparés par synthèse.
A titre de produits commerciaux dérivant des phospholipides, on peut plus particulièrement citer :

- l'EPICURON 120® (Lukas Meyer, Allemagne) qui est un mélange d'environ 70 % de phosphatidylcholine, 12 % de phosphatidyléthanolamine, et environ 15 % d'autres phospholipides ;
- l'OVOTINE 160® (Lukas Meyer, Allemagne) qui est un mélange comprenant environ 60 % de phosphatidylcholine, 18 % de phosphatidyléthanolamine, et 12 % d'autres phospholipides,
- un mélange de phospholipides purifiés à l'image des produits Lipoïd E75® ou Lipoïds E-80® (Lipoïd, Allemagne) qui est un mélange de phospholipides comprenant environ 80 % en poids de phosphatidylcholine, 8 % en poids de phosphatidyléthanolamine, 3,6 % en poids de lipides non polaires et 2% de sphingomyéline.

Selon un mode de réalisation préféré, le tensioactif lipophile est une lécithine dont la proportion en phosphatidylcholine varie de 40 à 80 % en poids.
Convient tout particulièrement comme source de phosphatidylcholine, le Lipoïd S75-3® (Lipoïd GmbH, Allemagne). Il s'agit de lécithine de soja. Cette dernière contient environ 69 % de phosphatidylcholine et 9 % de phosphatidyléthanolamine. Ce constituant est le seul constituant solide à 37 °C et à température ambiante dans la formulation. On peut également utiliser le polyglycéryl-6-dioléate (Plurol®).
Avantageusement, le/les tensioactif(s) précité(s) peuvent être associés à des co-tensioactifs comme par exemple d'autres phospholipides. A ce titre, les phosphatidylcholines (lécithine) sont particulièrement intéressantes.
D'autres phospholipides convenant à l'invention, peuvent être le phosphatidylglycérol, le phosphatidylinositol, la phosphatidylsérine, l'acide phosphatidique et la phosphatidyléthanolamine.
Avantageusement, selon une variante de réalisation, l'écorce lipidique solide est formée d'au moins un système tensioactif comprenant un tensioactif liposoluble tel que défini précédemment et un tensioactif thermosensible, hydrophile et non ionique.
Avantageusement, un tensioactif thermosensible, hydrophile et non ionique est hydrophile amphiphile.
Les tensioactifs émulsionnants habituellement utilisés ont un HLB (HLB = Hydrophilic Lipophilic Balance) allant de 8 à 18.
Ces tensioactifs peuvent être choisis parmi les alcools gras éthoxylés, les acides gras éthoxylés, les glycérides partiels d'acides gras éthoxylés, les triglycérides d'acides gras polyéthoxylés, et leurs mélanges.
Comme alcools gras éthoxylés, on peut citer par exemple les produits d'addition d'oxyde d'éthylène avec l'alcool laurylique, notamment ceux comportant de 9 à 50 groupes oxyéthylénés (Laureth-9 à Laureth-50 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétostéarylique (mélange d'alcool cétylique et d'alcool stéarylique), notamment ceux comportant de 9 à 30 groupes oxyéthylénés (Ceteareth-9 à Ceteareth-30 en noms CTFA) ; les produits d'addition d'oxyde d'éthylène avec l'alcool cétylique,

notamment ceux comportant de 9 à 30 groupes oxyéthylénés (Ceteth-9 à Ceteth-30 en noms CTFA) ; les produits d'addition d'oxyde d'éthylène avec l'alcool stéarylique, notamment ceux comportant de 9 à 30 groupes oxyéthylénés (Steareth-9 à Steareth-30 en noms CTFA) ; les produits d'addition d'oxyde d'éthylène avec l'alcool isostéarylique, notamment ceux comportant de 9 à 50 groupes oxyéthylénés (Isosteareth-9 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stéarate à PEG-50 stéarate) ; les palmito-stéarates de PEG-9 à PEG-50 ; les béhénates de PEG-9 à PEG-50 (en noms CTFA : PEG-9 béhénate à PEG-50 béhénate) ; et leurs mélanges.

On peut utiliser aussi des mélanges de ces dérivés oxyéthylénés d'alcools gras et d'acides gras.

Ces tensioactifs peuvent également être soit des composés naturels à l'image des phospholipides ou des composés synthétiques tels que les polysorbates qui sont des esters d'acide gras de sorbitol polyéthoxylé (Tween®), les esters de polyéthylène glycol d'acide gras provenant par exemple de l'huile de ricin (Crémophor®), des acides gras polyéthoxylés, par exemple de l'acide stéarique (Simulsol M-53®), des éthers d'alcool gras polyoxyéthylénés (Brij®), des éthers non phényles polyoxyéthylénés (Triton N®), des éthers hydroxylphényle polyoxyéthylénés (Triton X®).

Il peut plus particulièrement s'agir d'un 2-hydroxystéarate polyéthylène glycol et notamment celui commercialisé sous le nom Solutol® HS15 par la société BASF (Allemagne).

Selon un mode de réalisation préféré, l'écorce contient au moins un système tensioactif formé d'une lécithine et d'un 2-hydroxystéarate polyéthylène glycol et notamment celui commercialisé sous la dénomination Solutol® HS 15.

Selon une première variante de réalisation, au moins un tensioactif précité est, sous réserve qu'il possède une fonction ester, impliqué par ailleurs dans au moins une réaction de transacylation considérée selon l'invention.

Selon une seconde variante de réalisation, l'écorce lipidique comprend, outre au moins un des tensioactifs précités, au moins un ester d'alcool distinct desdits tensioactifs, présent pour assurer au moins une réaction de transacylation requise selon l'invention.

Avantageusement, le nombre de carbone dudit ester d'alcool est un entier inférieur ou égal à 12, plus particulièrement inférieur ou égal à 10, encore plus particulièrement inférieur ou égal à 8.

De tels esters sont illustrés dans l'exemple 6.

Parmi les esters d'alcool convenant à la présente invention, l'ester répondant à la formule suivante est particulièrement intéressant :

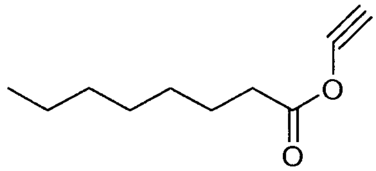

L'incorporation de ces esters dans une formulation de nanocapsules lipidiques selon l'invention peut se faire dans une proportion pondérale de l'ordre de 1 à 25% en poids et plus particulièrement de 5 à 20% en poids par rapport au poids total du mélange des tensioactif(s) et des esters considérés pour la formation de l'écorce lipidique.

D'une manière générale, des capsules conformes à l'invention peuvent contenir de l'ordre de 1 à 10 % en poids d'esters d'alcool distincts des tensioactifs qu'elles contiennent par ailleurs, par rapport à leur poids total, en ne tenant en compte que les substances inorganiques de la formulation.

L'emploi d'ester d'alcool selon la présente invention permet de renforcer significativement la stabilité des propriétés initiales des LNCs, notamment au niveau de leur stabilité colloïdale et de leur furtivité. Un autre avantage, conféré par l'utilisation de tels esters d'alcool à titre de sites pour la réaction de transacylation au niveau de l'écorce lipidique des capsules selon la présente invention, repose sur leur grande accessibilité aux réactifs potentiellement utilisables lors de cette étape de transacylation, avec comme résultat une amélioration du rendement de fixation, comme on peut le voir dans les exemples.

Bien entendu, les deux variantes de réalisation de réaction de transacylation précitées peuvent coexister dans une capsule selon l'invention sous réserve bien sûr que l'écorce de celle-ci contienne, outre les tensioactifs notamment lipophiles, au moins un ester d'alcool tel que défini ci-dessus.

L'écorce peut, le cas échéant, encapsuler en outre au moins un actif. Dans ce cas, il s'agira plutôt d'un actif de nature liposoluble ou lipodispersible.

Avantageusement, de tels actifs liposolubles ou lipodispersibles sont déjà présents dans les nanocapsules de départ, mises en oeuvre en étape i) du procédé de préparation selon l'invention.

Selon un mode de réalisation, l'écorce lipidique solide est dénuée de protéines.

Les capsules selon la présente invention comportent par ailleurs un coeur lipidique liquide.

Coeur lipidique liquide

Le coeur lipidique liquide comprend au moins une phase grasse huileuse formée d'au moins un corps gras liquide ou semi-liquide, et en particulier d'au moins un triglycéride, d'un ester d'acide gras, ou d'un de leurs mélanges.

L'ester d'acide gras peut être plus particulièrement choisi parmi les esters d'acides gras en $C_8$ à $C_{18}$, notamment en $C_8$ à $C_{12}$, et notamment le palmitate d'éthyle, l'oléate d'éthyle, le myristate d'éthyle, le myristate d'isopropyle, le myristate d'octydodécyle et leurs mélanges.

Les triglycérides mis en oeuvre peuvent être des triglycérides de synthèse ou des triglycérides d'origine naturelle. Les sources naturelles peuvent inclure les graisses animales ou les huiles végétales par exemple les huiles de soja ou les sources en triglycérides à longue chaîne (LCT).

D'autres triglycérides d'intérêt sont composés principalement d'acides gras de longueurs moyennes encore appelés triglycérides à chaîne moyenne (MCT). Une huile à triglycérides à chaîne moyenne (MCT) est un triglycéride dans lequel la chaîne carbohydrate a de 8 à 12 atomes de carbone.

De telles huiles MCT sont disponibles commercialement.

A titre d'exemple de ces huiles MCT, on peut citer les TCR (nom commercial de la société industrielle des oléagineux, France, pour un mélange de triglycérides dans lequel environ 95 % des chaînes d'acides gras possèdent 8 ou 10 atomes de carbone) et le Miglyol® 812 (triglycéride commercialisé par la société Dynamit Nobel Suède, pour un mélange de triesters de glycérides d'acides caprylique et caprique).

Les motifs d'acides gras de ces triglycérides peuvent être insaturés, monoinsaturés ou polyinsaturés. Les mélanges de triglycérides ayant des motifs d'acides gras variables sont également acceptables.

L'indice HLB ou balance hydrophile-lipophile est tel que défini par C. Larpent dans le Traité K.342 des Editions Techniques de l'Ingenieur.

Convient tout particulièrement à l'invention, le triglycéride commercialisé sous le nom Labrafac WL 1349®.

Dans un mode de réalisation préféré, la phase grasse est un triglycéride d'acide gras.

Comme indiqué précédemment, le coeur lipidique liquide peut, le cas échéant, encapsuler en son sein un actif. Cet actif est de préférence liposoluble. Toutefois, des actifs de nature hydrosoluble ou hydrodispersible peuvent également être encapsulés dans le coeur lipidique. Dans ce cas, il devra subir une formulation préalable à son encapsulation.

Par exemple, l'actif pourra se présenter sous la forme de micelles inverses ou de microémulsions, comme cela est par exemple décrit dans les documents WO 09/001019 et FR 2 916 974, respectivement.

A titre illustratif et non limitatif d'actifs pouvant être encapsulés selon l'invention, on peut notamment citer la doxorubicine et leurs sels d'addition à un acide pharmaceutiquement acceptable et plus particulièrement le chlorhydrate, et des héparines de bas poids moléculaire.

**Procédé de préparation de capsules selon l'invention**

Comme indiqué précédemment, des capsules à l'état nanoparticulaire subissent une réaction de transacylation, entre au moins un constituant de leur enveloppe lipidique solide et au moins un composé aminé.

Cette réaction de transacylation correspond à une réaction d'échanges de groupement(s) acyle(s) entre un composé de l'enveloppe lipidique, de préférence un tensioactif, en particulier un tensioactif liposoluble, ou un ester d'alcool, si présent, tels que décrits ci-dessus, et le composé aminé considéré.

Selon une variante de réalisation préférée, ledit tensioactif liposoluble est une lécithine.

Avantageusement, une telle réaction est simple d'exécution et ne requiert pas de manipulation délicate.

La première étape de cette réaction consiste en une alcalinisation de la surface des nanocapsules pour les activer, vis-à-vis d'une réaction de transacylation, par exemple, par contact de ces nanocapsules avec au moins une solution aqueuse alcaline.

Avantageusement, la quantité d'agent alcalin à ajouter à la phase aqueuse dans laquelle on disperse directement les nanocapsules, pour déclencher la réaction de transacylation, est telle que le pH de la suspension aqueuse de nanocapsules est compris entre 8 et 14, et encore de préférence entre 8 et 10.

L'agent alcalin à ajouter pour déclencher la réaction de transacylation peut être par exemple choisi parmi la soude, la potasse ou un composé aminé tel que par exemple la la triéthylamine.

La deuxième étape de cette réaction consiste en la mise en présence de ces nanocapsules alcalinisées avec au moins un composé aminé dans des conditions propices à la transacylation. La réaction est laissée de préférence sous agitation le temps nécessaire au déroulement de la réaction, c'est-à-dire entre 5 minutes et 30 minutes.

Par exemple, le temps pendant lequel les particules sont maintenues au sein de la solution alcaline pour que se développe la réaction de transacylation peut être compris entre 5 min et 1 heure, de préférence entre 5 min et 30

min, de préférence encore, il est de 15 min.

Ledit composé aminé employé dans la réaction de transacylation fonctionnalisant les nanocapsules selon la présente invention est avantageusement tel que défini précédemment.

Le rendement de la réaction de transacylation peut être accru par augmentation de la durée de ladite réaction de transacylation et/ou par des variations de la composition de la solution alcaline déclenchant ladite réaction de transacylation, et en particulier, par augmentation de la quantité d'agent alcalin utilisé pour préparer ladite solution alcaline.

Avantageusement, il est réalisé, à l'issue de la transacylation, une dernière étape de neutralisation du milieu réactionnel par ajout d'une solution d'acide chlorhydrique de concentration adaptée, généralement comprise entre 0,1 mol/litre et 6 mol/litre, le pH devant être ramené à une valeur comprise entre 7 et 7,4.

L'agent acide utilisé pour neutraliser la suspension aqueuse de particules après la réaction de transacylation peut être par exemple choisi parmi les acides organiques monocarboxyliques ou polycarboxyliques, porteurs ou non de fonctions alcool, comme l'acide acétique, l'acide citrique, l'acide tartrique, l'acide succinique, l'acide malique, l'acide lactique ou un acide minéral comme l'acide chlorhydrique ou l'acide sulfurique.

Ce temps de neutralisation des particules, c'est-à-dire le temps d'agitation nécessaire après ajout de l'acide au milieu réactionnel peut être compris entre 5 min et 1 heure, de préférence entre 5 min et 30 min, de préférence encore, il est de 15 min.

Les nanocapsules modifiées peuvent être ensuite purifiées selon les méthodes de purification bien connues de l'homme de l'art telle que la technique de dialyse, filtration ou les techniques chromatographiques (HPLC par exemple, colonne échangeuses d'ions, séparation par exclusion stérique).

**<u>Procédé de préparation des nanocapsules de départ</u>**

Au sens de l'invention le terme nanocapsules est à distinguer de nanosphères. On entend par nanocapsules des particules constituées d'un coeur liquide ou semi-liquide à température ambiante, enrobé d'un film ou écorce solide à température ambiante, par opposition à des nanosphères qui sont des particules matricielles, i.e. dont la totalité de la masse est solide. Ainsi, lorsque les nanosphères contiennent un principe pharmaceutiquement actif, celui-ci est finement dispersé dans la matrice solide.

Avantageusement, les nanocapsules de l'étape i) du procédé selon l'invention, c'est-à-dire les nanocapsules à un état non fonctionnalisé, c'est-à-dire n'ayant pas subies de transacylation, possèdent une taille moyenne inférieure à 150 nm, de préférence inférieure à 100 nm, de préférence encore inférieure à 50 nm. Ces tailles peuvent être déterminées par spectroscopie à corrélation de photons, microscopie électronique à balayage, microscopie électronique à transmission en mode cryoscopique.

L'épaisseur du film ou écorce solide est avantageusement comprise entre 2 à 10 nm. Elle est égale environ au dixième du diamètre des particules. Cette épaisseur peut être calculée par bilan de masse, ou visualisée par microscopie électronique à transmission par ombrage négatif ou alors par microscopie électronique à transmission en mode cryoscopique ou par diffusion des neutrons aux petits angles.

Compte-tenu de leur taille, les nanocapsules de départ sont des particules lipidiques colloïdales.

L'indice de polydispersité des nanocapsules de l'invention est avantageusement compris entre 5 et 15 %. Cet indice est déterminé sur l'histogramme de taille obtenu par la méthode de spectroscopie à corrélation de photons.

Les nanocapsules sont chacune constituées d'un coeur essentiellement lipidique liquide ou semi-liquide à température ambiante, enrobé d'une écorce essentiellement lipidique solide à température ambiante.

Au sens de l'invention, l'expression « essentiellement lipidique » signifie que le noyau et l'écorce formant les nanocapsules selon l'invention sont constituées à plus de 50 % en poids, en particulier à plus de 75 % en poids, notamment à plus de 80 % en poids, voire plus de 90 %, plus particulièrement plus de 95 % de leurs poids respectifs, voire en totalité d'un ou plusieurs composés lipidiques (hydrophobes). Ces pourcentages sont appréciés en considérant comme faisant partie de la phase liquide les tensioactifs qui sont présents.

Au sens de l'invention, l'expression température ambiante désigne une température variant de 18 à 25 °C.

Les nanocapsules de départ peuvent avantageusement être obtenues selon un procédé comprenant au moins les étapes consistant à :

a) disposer d'une microémulsion formulée par inversion de phase d'une émulsion et stabilisée par au moins un système tensioactif contenant au moins un tensioactif liposoluble tel que défini précédemment,

b) effectuer la trempe de ladite microémulsion de manière à obtenir des nanocapsules formées d'un coeur lipidique, liquide à température ambiante et enrobé d'un film lipidique solide à température ambiante.

Une microémulsion convenant tout particulièrement à la formation de nanocapsule de départ comprend au moins une phase grasse huileuse, une phase aqueuse et un système tensioactif comprenant au moins un tensioactif lipophile tels que définis précédemment, et de préférence, en outre, un tensioactif thermosensible, hydrophile et non

ionique, et, le cas échéant, au moins un ester d'alcool, dont le nombre de carbone est préférentiellement un entier inférieur ou égal à 12, plus particulièrement inférieur ou égal à 10, encore plus préférentiellement inférieur ou égal à 8.

Une telle microémulsion peut par exemple être préparée de la façon suivante.

L'ensemble des constituants destinés à former la microémulsion est pesé dans un récipient. Le mélange est homogénéisé, par exemple au moyen d'un Rayneri 350 tours/min, et chauffé en augmentant progressivement la température au moyen d'un bain-marie jusqu'à une température supérieure ou égale à la température d'inversion de phase $T_2$, c'est-à-dire jusqu'à l'obtention d'une phase blanche plus visqueuse qui indique l'obtention de l'émulsion inverse. Le chauffage est alors stoppé et l'agitation maintenue jusqu'à retour à la température ambiante, en passant par la température d'inversion de phase $T_1$, c'est-à-dire la température à laquelle se forme la microémulsion attendue, sous la forme d'une phase transparente ou translucide. Il est à noter que lorsque la température est redescendue en dessous de la zone d'inversion de Phase en Température ($T_1$), on obtient à nouveau l'émulsion de départ. Cette succession d'opération est avantageusement répétée et lorsque l'on est à nouveau à la température $T_1$ d'inversion de phase, on refroidit pour former les nanocapsules attendues.

Une telle technique est plus particulièrement décrite dans les documents FR 2 916 974 et EP 1 265 698 précités.

Dans le cas où il est souhaité d'encapsuler un actif notamment de nature lipophile au sein du coeur lipidique, de telles nanocapsules de départ peuvent être obtenues selon un procédé de préparation similaire, comprenant deux étapes supplémentaires entre les étapes a) et b) précitées, consistant à :

- disposer d'une seconde composition, distincte de ladite microémulsion et formée en tout ou partie d'au moins un actif,
- mettre en présence ladite microémulsion avec ladite seconde composition dans des conditions propices à l'interaction dudit actif avec ladite microémulsion.

Un tel procédé est plus particulièrement détaillé dans FR 2 916 974.

Alternativement, de telles nanocapsules de départ encapsulant un actif peuvent être préparées selon un procédé de préparation comprenant au moins les étapes consistant à :

- disposer d'au moins une première microémulsion à caractère eau-dans-huile, stabilisée par au moins un tensioactif lipophile tel que défini précédemment et contenant au niveau de sa phase hydrophile au moins un actif, notamment à caractère hydrophile ou hydrodispersible tel que défini précédemment,
- disposer d'au moins une seconde microémulsion, distincte de la première microémulsion, formulée par inversion de phase d'une émulsion et stabilisée par au moins un tensioactif thermosensible, non ionique et hydrophile tel que défini précédemment,
- ajouter ladite première microémulsion dans ladite seconde microémulsion, dans des conditions propices à la formation d'une nouvelle microémulsion internalisant ledit actif au niveau de sa phase hydrophile, et
- effectuer la trempe de ladite microémulsion obtenue à l'étape précédente, de manière à obtenir les nanocapsules attendues.

Selon une variante de réalisation, au moins l'une des microémulsions, et de préférence la première, contient en outre au moins un ester d'alcool distinct des tensioactifs.

La présente invention est illustrée par les exemples et figures suivants qui sont présentés à titre illustratif et non limitatif du domaine de l'invention.

**Figures :**

- Figure 1 : Evolution de la taille (en nm) des nanocapsules obtenues selon le procédé de préparation de l'invention, en fonction du volume de NaOH (1N) ajouté (en µl), en présence d'une quantité constante de sérum albumine humaine.
- Figure 2 : Evolution de la taille (en nm) des nanocapsules obtenues selon le procédé de préparation de l'invention, en fonction du volume de NaOH (10N) ajouté (en µl), en présence d'une quantité constante de sérum albumine humaine.
- Figure 3 : Evolution de la taille (en nm) des nanocapsules obtenues selon le procédé de préparation de l'invention, en fonction du temps de réaction et pour un volume de NaOH (10N) fixe, de 500 µl), en présence d'une quantité constante de sérum albumine humaine.
- Figure 4 : Evolution du potentiel zeta (en mV) des nanocapsules obtenues selon le procédé de préparation de l'invention, en fonction du volume de polylysine ajouté (en µl).
- Figure 5 : Evolution de la viabilité cellulaire relative (en %) sur une lignée de cellules U87MG en fonction de la

concentration en nanocapsules, modifiées ou non, obtenues selon le procédé de préparation de l'invention.

- Figure 6 : Evolution du rapport $I/I_0$ en fonction de la concentration en LNCs modifiées par transacylation, I représentant l'intensité de fluorescence de complexes fluorescents BSA-FITC adsorbées sur ces LNCs, tandis que $I_0$ représente l'intensité de fluorescence de la BSA-FITC libre en solution.
- Figure 7 : Evolution du rapport $I/I_0$ en fonction de la concentration en LNCs modifiées par transacylation, I représentant l'intensité de fluorescence de complexes fluorescents ARNsi-FITC adsorbées sur ces LNCs, tandis que $I_0$ représente l'intensité de fluorescence des ARNsi-FITC libres en solution.

**Exemple 1 : Préparation de nanocapsules fonctionnalisées par de l'albumine sérique humaine**

1. Préparation d'une microémulsion non chargée en actif

On réalise 5 g d'une émulsion contenant 75 mg de Lipoïd S75-3®, 504 mg de Labrafac WL 1349® lipophile, 504 mg de Solutol HS®, 15,383 g d'eau et 88 mg de chlorure de sodium.

L'ensemble est réuni dans un même bêcher et placé sous agitation magnétique. On chauffe jusqu'à atteindre une température de 85 °C. Toujours sous agitation magnétique, on laisse refroidir le système jusqu'à une température de 60 °C. Ces cycles thermiques (entre 85 °C et 60 °C) sont réalisés trois fois de façon à obtenir des microémulsions de mieux en mieux structurées. Le système est alors maintenu sous sa forme microémulsion en le stabilisant à une température comprise dans (ou au proche voisinage) de la Zone d'Inversion de Phase, en l'espèce 65 °C.

2. Obtention de LNC

La microémulsion stabilisée à la température d'inversion de phase est refroidie soit brutalement soit diluée avec de l'eau froide de manière à former une suspension de LNC.

3. Fixation de l'albumine sérique humaine

10 mL de nanocapsules dites LNC sont incubés pendant 10 minutes, sous agitation, en présence de NaOH (1N) (volume variable de 0 à 2000μl). Ensuite, on ajoute 500 μl d'une solution de sérum albumine humaine à 5 % en masse. On laisse réagir 15 minutes, puis on neutralise la solution avec une solution d'acide chlorhydrique de concentration égale à 1 M, le volume dépendant du volume de soude utilisée au départ. Les résultats obtenus sont indiqués en Figure 1.

**Exemple 2 : Préparation de nanocansules fonctionnalisées par de l'albumine sérique humaine**

Les LNC fonctionnalisées sont préparées par analogie avec le procédé décrit en exemple 1, en utilisant cette fois une solution de NaOH (10N).

Les résultats obtenus sont indiqués en Figure 2.

**Exemple 3 Préparation de nanocansules fonctionnalisées par de l'albumine sérique humaine**

Les LNC fonctionnalisées sont préparées par analogie avec le procédé décrit en exemple 2, en faisant varier cette fois le temps de réaction de 0 à 30 minutes.

Les résultats obtenus sont indiqués en Figure 3.

**Exemple 4: Préparation de nanocapsules fonctionnalisées par de la polylysine FITC**

On procède par analogie avec le mode de préparation de l'exemple 1, en faisant cette fois varier le volume de polylysine ajouté de 0 à 200 μl. On mesure le potentiel zeta en fonction du volume de polylysine ajouté.

Les résultats obtenus sont indiqués en Figure 4.

Il apparaît très clairement une dépendance entre le potentiel zeta des nanocapsules lipidiques modifiées et la quantité de polylysine introduite dans le mélange réactionnel, c'est-à-dire une relation entre la quantité de polylysine fixée sur les nanocapsules lipidiques et le potentiel zeta de ces dernières, ce qui conditionne leur aptitude à fixer des molécules hydrophiles chargées négativement.

De plus, il est possible de suivre au fur et à mesure des étapes de production la quantité de protéines fixée sur les nanocapsules lipidiques.

Le tableau I ci-dessous présente les résultats de dosage des protéines avant et après l'étape de dialyse. Le dosage est basé sur la méthode μBCA.

Le μBCA est une méthode de dosage spectrophotométrique, basée sur l'absorption de la lumière par un

complexe formé par les protéines à doser et le réactif µBCA. Cette méthode requiert l'utilisation d'une gamme étalon réalisée à partir d'une solution de protéines de concentration connue (µBCA de PIERCE).

**Tableau I**

| Echantillons n° | Concentration en protéines avant dialyse en µg par mL | Echantillons n° | Concentration en protéines après dialyse en µg par mL |
|---|---|---|---|
| 1 | 184,28 | 1 | 310 |
| 2 | 371,42 | 2 | 281,42 |
| 3 | 601,42 | 3 | 448,57 |
| 4 | 642,85 | 4 | 622,85 |
| 5 | 1228,57 | 5 | 530 |
| 6 | 1002,85 | 6 | 504,28 |
| 7 | 1274,28 | 7 | 982,85 |

**Exemple 5: Utilisation de nanocapsules fonctionnalisées par de la polylysine FITC pour la complexation d'ARNsi**

Les nanocapsules fonctionnalisées par de la polylysine FITC sont préparées par analogie avec le mode de préparation de l'exemple 1 en présence de 0,5 µl de LNCs de 50 nm et 400 µl de PLL-FITC. L'ensemble est dialysé, filtré et son pH ajusté à 7,4.

Ces particules fonctionnalisées par de la polylysine FITC sont ensuite mises en présence avec différents volumes (2, 3, 4, 5, 6, 7, 8, 9, 10, 12, 15 et 20 µl) d'ARNsi (20 pb soit 16 000 Daltons) de concentration 0,001 µmol/ml.

L'ensemble des mélanges ainsi préparés est caractérisé sur gel électrophorétique.

A des fins comparatives, il est également réalisé le gel électrophorétique de l'ARNsi seul, et celui d'un standard de PCR 20 pb représentatif de bas poids moléculaire.

On note que, pour tous les mélanges formés à partir de, respectivement, de 2 µl à 15 µl de la solution d'ARNsi, un unique spot figure sur le gel électrophorétique, correspondant à l'ARNsi complexé.

En revanche, au-delà de 15 µl, on ne constate plus qu'une complexation partielle de l'ARNsi. Il apparaît donc qu'au-delà de 15 µl d'ARNsi (0,001 µmole/ml) la capacité de fixation des LNC est dépassée.

**Exemple 6 : Préparation de nanocapsules fonctionnalisées par des esters d'alcools**

1) Synthèse des esters

Deux esters d'alcool, ester 1 et ester 2, ont été synthétisés et présentent les structures chimiques suivantes :

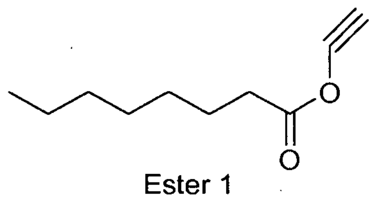

Ester 1

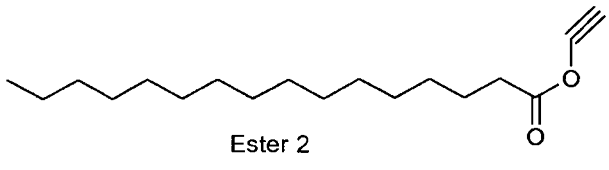

Ester 2

Ces deux esters sont synthétisés selon le protocole suivant.

Dans un ballon tricol de 250 ml, est d'abord introduit 0,1 mole, respectivement, de chlorure d'octanoyle (Ref. 04733, Sigma-Aldrich) ou de chlorure de palmitoyle (Ref. P78, Sigma-Aldrich), puis 150 ml d'éther diéthylique (Ref. 346136, Sigma-Aldrich). Après agitation magnétique, le mélange est refroidi à l'aide d'un bain d'eau glacée avant l'introduction successive de 0,15 mole d'alcool puis de 0,15 mole de triéthylamine (Ref. T0886, Sigma-Aldrich).

Après retrait du bain d'eau glacée, le mélange est porté à reflux pendant 2 heures.

Après arrêt du chauffage et refroidissement, le mélange réactionnel est filtré sur papier filtre avant de procéder à une extraction liquide-liquide à l'aide de dichlorométhane (Ref. 443484, Sigma-Aldrich).

La phase organique est lavée successivement avec de l'eau (deux fois 500 ml) puis une solution saturée de chlorure de sodium. La phase organique est ensuite séchée à l'aide de sulfate de sodium anhydre, puis mise sous pression réduite pour l'évaporation des solvants.

Le produit désiré est obtenu sous forme d'un liquide incolore (ester 1) ou d'un solide cristallin blanc (ester 2). Après séchage du produit sous pression réduite, celui-ci est pesé et le rendement de la réaction est calculé.

Ce dernier est compris entre 90 et 95%.

2) <u>Obtention de LNCs chargées en esters</u>

Des nanocapsules non chargées en actifs ont été obtenues par le procédé décrit en exemple 1, l'émulsion comprenant également cette fois une quantité d'ester 1, d'ester 2 ou de Span 40®, à hauteur de 10 ou 20 % en masse de la quantité de tensioactifs présents dans l'émulsion (Solutol HS-15 et Lipoïd S75-3).

Le Span 40® (Ref 85545, Fluka) est un ester de formule suivante :

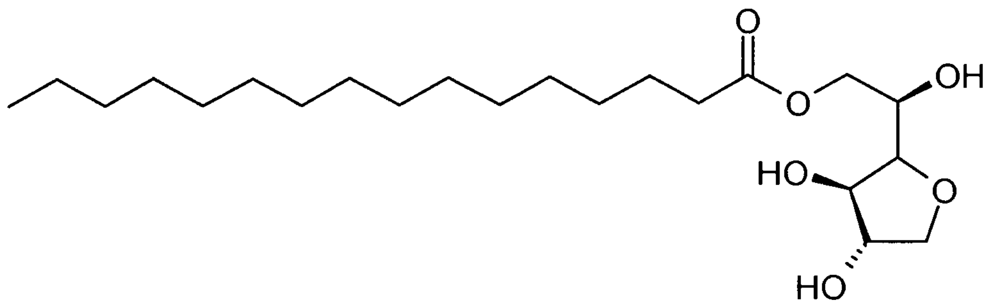

3) <u>Fixation de l'albumine sérique humaine</u>

Les LNCs sont fonctionnalisées suivant le protocole décrit dans l'exemple 1.

4) <u>Résultats</u>

Les caractéristiques physico-chimiques des nanocapsules lipidiques ainsi produites sont consignées dans le Tableau II suivant :

**<u>Tableau II</u>**

| Nom de l'échantillon | Diamètre hydrodynamique en nm (*) | Potentiel zeta en mV (**) |
|---|---|---|
| LNCs 50nm + 10% ester 2 | 30,1 | -15 |
| LNCs 50nm + 20% ester 2 | 32 | -12 |
| LNCs 50nm + 10% ester 2 transacylation PEI | 40,1 | 25,5 |
| LNCs 50nm + 20% ester 2 transacylation PEI | 44,1 | 37,3 |
| LNCs 50nm + 10% ester 1 | 31,3 | -23,9 |
| LNCs 50nm + 20% ester 1 | 35,2 | -29,3 |
| LNCs 50nm + 10% ester 1 transacylation PEI | 52 | 34,3 |
| LNCs 50nm + 20% ester 1 transacylation PEI | 54,5 | 38,7 |
| LNCs 50nm + 10% Span 40 | 52 | -7 |
| LNCs 50nm + 20% Span 40 | 85 | 42 |
| LNCs 50nm + 10% Span 40 transacylation PEI | 48 | -4 |

(suite)

| Nom de l'échantillon | Diamètre hydrodynamique en nm (*) | Potentiel zeta en mV (**) |
|---|---|---|
| LNCs 50nm + 20% Span 40 transacylation PEI | 90,2 | 24,8 |
| (*) et (**) : ces valeurs ont été mesurées à l'aide d'un NanoZS (Malvern Instruments). | | |

**Exemple 7 : Mesure du rendement de transacylation en présence d'esters**

Après purification, un dosage des réactifs de transacylation a été réalisé afin de visualiser l'augmentation du rendement de transacylation lié à l'emploi des esters précédemment cités.

La méthode de dosage repose sur l'emploi de l'o-phthaldialdéhyde (OPA) comme réactif, et se déroule selon l'équation bilan suivante :

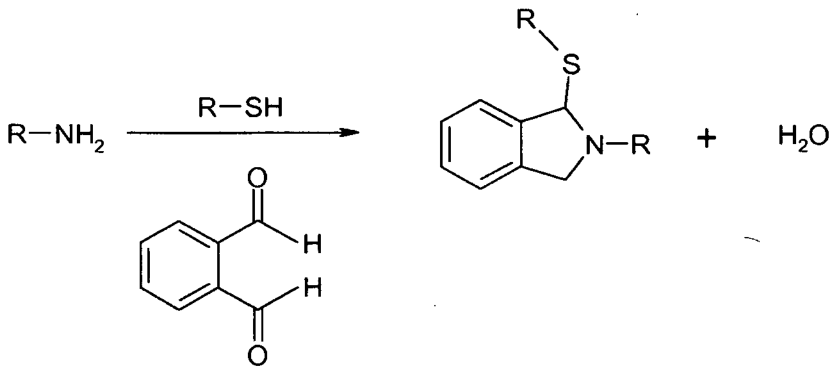


Les dérivés indoles produits sont fluorescents et permettent de quantifier la totalité des groupes amines primaires présents dans l'échantillon de nanocapsules.

Tout d'abord, une gamme étalon de la substance à doser est réalisée. Pour ce faire, la substance est dissoute à différentes concentrations (50, 100, 150 et 200 μg/ml) dans l'eau à pH 7,4. La fluorescence de ces solutions est évaluée à 460 nm, pour une longueur d'onde d'excitation de 355 nm.

La substance d'intérêt est ensuite dosée dans les échantillons de nanocapsules lipidiques en ajoutant 300 μl d'OPA à 30 μl de solution de LNCs.

La fluorescence de cette solution est mesurée à 460 nm et la concentration en substance est calculée à l'aide de la gamme étalon.

Cette méthode de dosage a été utilisée avec succès pour doser le réactif greffé sur les nanocapsules lipidiques lors de l'étape de transacylation. Pour chaque réactif (poly-L-lysine (PLL) et polyéthylène imine (PEI)), une gamme-étalon spécifique a été utilisée. Les résultats du dosage, réalisé après purification de l'échantillon, sont consignés dans le Tableau III suivant :

**TABLEAU III**

| Nom de l'échantillon | Concentration en polymère μg/ml | Rendement de fixation en % |
|---|---|---|
| LNCs 50nm transacylation Poly-L-lysine | 0,98 | 0,98 |
| LNCs 50nm + 10% ester 1 transacylation PEI | 15 | 15 |
| LNCs 50nm + 20% ester 1 transacylation PEI | 23 | 23 |
| LNCs 50nm + 10% ester 2 transacylation PEI | ND | ND |
| LNCs 50nm + 20% ester 2 transacylation PEI | 1,6 | 1,6 |
| LNCs 50nm + 10% Span 40 transacylation PEI | 1,06 | 1,06 |
| LNCs 50nm + 20% Span 40 transacylation PEI | 2,53 | 2,53 |

Le rendement de fixation correspond au rapport entre la quantité de substance greffée sur les LNCs (CR) sur la quantité théorique maximale de cette substance (CT).

La substance greffée est dosée par la méthode OPA afin d'obtenir le CR. Le rendement de fixation R peut alors s'écrire :

$$R = (CR/CT)*100$$

Comme on peut l'observer dans ce tableau, lorsque la réaction de transacylation est produite sur des nanocapsules lipidiques classiques (LNCs 50 nm transacylation poly-L-lysine), le rendement de fixation est proche de 1 %.

L'introduction de 10 ou 20% de l'ester 2 ou de Span 40 dans la formulation permet de multiplier ce rendement par un facteur compris entre 2 et 3.

L'introduction de 10 ou 20 % de l'ester 1 dans la formulation entraîne quant à elle une augmentation du rendement par un facteur compris entre 15 et 25.

**Exemple 8 : Evaluation de la toxicité des nanocapsules obtenues par le procédé de transacylation**

La toxicité des nanocapsules lipidiques modifiées par le procédé de transacylation a été évaluée en utilisant le test au MTT (Bromure de 3-(4,5-diméthylthiazol-2-yl)-2,5-diphényl tetrazolium).

Ce test vise à mesurer la toxicité des nanoparticules en mesurant la viabilité relative des cellules exposées aux nanoparticules en prenant comme témoin des cellules non traitées par des nanoparticules.

L'anneau de tétrazolium que contient le réactif MTT est réduit en formazan par la succinate déshydrogénase mitochondriale des cellules vivantes actives.

La couleur du milieu passe alors du jaune au bleu-violacé, l'intensité de cette coloration étant proportionnelle au nombre de cellules vivantes présentes lors du test mais aussi à leur activité métabolique.

Pour ce test, on peut se référer aux tests décrits dans les documents « Mosmann T, Journal of immunological methods 65 (1-2) : 55-63, December 1983 », et « Cory et al, Cancer communications 3 (7) : 207-12, July 1991 ».

Ce test a été réalisé sur une lignée cellulaire humaine et tumorale, des cellules U87MG (HTB-14, LGC standard).

Pour assurer une bonne significativité statistique du test, chaque essai a été réalisé en quintuple.

Les résultats obtenus sont indiqués en Figure 5.

Pour chaque graphique, la fiabilité cellulaire relative est exprimée en fonction de la concentration des nanocapsules modifiées ou non. On utilise comme référence des cellules U87MG non traitées par des nanoparticules. La valeur obtenue pour ces cellules représente une fiabilité cellulaire de 100 %.

On peut observer que, d'une manière générale, les nanocapsules modifiées lors de l'étape de transacylation présentent des profils de toxicité comparables au profil des nanocapsules de départ.

Au vu des résultats obtenus, le greffage de polymères naturels ou synthétiques n'entraîne pas de toxicité additionnelle.

En effet, il a été observé que les nanocapsules obtenues par transacylation ne montrent aucune toxicité pour des concentrations inférieures à 1/300 de la suspension initiale.

**Exemple 9: Développement de structures en multicouches autour de nanocapsules lipidiques modifiées par transacylation**

Sur 2 ml de suspension de LNCs de 50 nm obtenues selon le procédé décrit dans l'exemple 1, une transacylation est réalisée à 25°C par utilisation de 40 µl d'une solution à 5% de poly-L-lysine (PLL) pendant 15 minutes. Le mélange est ensuite neutralisé par ajout de 2 ml d'un tampon glycine 0,5M à pH 2,2.

Après dialyse contre de l'eau milliQ, 50 µl de LNCs transacylées sont mis en contact avec 0,045 nanomoles d'ARNsi, puis diluées avec 2,95 ml d'eau milliQ. L'intégralité des ARNsi se fixe alors sur les LNCs. La concentration en ARNsi est de 15 nanomoles par litre.

Il en suit une augmentation du diamètre hydrodynamique des particules, ainsi qu'une inversion de leur potentiel zeta, confirmant ainsi l'adsorption des ARNsi sur les LNCs.

Une couche de polymères (poly-L-lysine) est consécutivement adsorbée sur les LNCs selon le protocole suivant.

5 µl de la solution de polylysine à 5% sont ajoutés à la suspension de LNCs-ARNsi pour former des systèmes LNCs-ARNsi-PLL, qui peuvent alors être purifiés par dialyse contre une solution de NaCl 10 mM.

Les LNCs ainsi obtenues ont un potentiel zeta positif, témoignant ainsi de l'adsorption du polymère dans une structure multicouches.

**Exemple 10 : Fixation de protéines modèles sur des nanocapsules modifiées par transacylation**

Cette étude consiste en l'adsorption de protéines ou d'acides nucléiques fluorescents sur les LNCs modifiées.

1) Protéine fluorescentes BSA-FITC

Des expériences ont été réalisées avec de la BSA-FITC à différentes concentrations : 0,5 mg/ml, 0,33 mg/ml, 0,25 mg/ml, 0,2 mg/ml, et 0,1 mg/ml.

Ces protéines marquées au FITC ont été obtenues selon le protocole suivant. 900 µl d'une solution de BSA à 2,77 mg/ml dans du bicarbonate de sodium 0,1M à un pH égal à 9, sont mélangés à 250 µl d'une solution de FITC à 4 mg/ml dans du diméthylsulfoxide, la réaction étant laissée sous agitation à l'abri de la lumière à 25°C pendant 2 heures. On sépare ensuite les protéines marquées, du FITC n'ayant pas réagi, par chromatographie d'exclusion stérique sur gel de Sephadex G-25®.

La quantité de protéine ainsi que la quantité de FITC fixée sur les protéines sont ensuite dosées selon le protocole suivant.

Une gamme étalon de la BSA et une gamme étalon du FITC sont réalisées dans du PBS 5 mM, l'absorbance étant mesurée à 280 nm pour la BSA, à 490 nm pour le FITC.

Les absorbances à 280 nm et 490 nm de la solution de protéines marquées sont ensuite mesurées et comparées aux gammes étalons afin de déterminer les concentrations respectives en BSA et en FITC. Le rapport concentration en FITC sur concentration en BSA peut alors être calculé.

Il est ensuite procédé à l'étape de complexation entre LNCs transacylées et protéines fluorescentes selon le protocole suivant.

Pour obtenir les valeurs de fluorescences $I_0$, la fluorescence et les intensités de fluorescence de solutions de BSA-FITC de concentrations connues sont mesurées. Puis les mêmes solutions de BSA-FITC sont réalisées en présence de concentrations croissantes de nanocapsules transacylées. Les fluorescences et intensités de fluorescence de ces solutions sont alors également mesurées (I).

En comparant le rapport $(I-I_0)/I_0$ pour chaque concentration en LNCs modifiées, on observe une diminution de l'intensité de fluorescence avec l'augmentation de la concentration en LNCs modifiées.

Les résultats de mesure de fluorescence obtenus sont représentés Figure 6.

Lorsque des expériences similaires ont été réalisées avec des LNCs non préalablement transacylées, aucune variation de l'intensité de fluorescence n'a été observée.

2) ARNsi-FITC

Les mêmes expériences ont été menées avec, à la place de la BSA-FITC, des ARNsi-FITC. Une même diminution de l'intensité de fluorescence avec l'augmentation de la concentration en LNCs modifiées est observée.

Les résultats de mesure de fluorescence obtenus avec les ARNsi-FITC sont représentés Figure 7.

## Revendications

1. Capsule de type coeur lipidique liquide/écorce lipidique solide fonctionnalisée en surface avec au moins un composé contenant au moins une fonction amine, **caractérisée en ce que** l'architecture coeur/écorce lipidiques est à l'échelle nanométrique et **en ce que** ledit composé est lié de manière covalente via une réaction de transacylation à ladite écorce lipidique solide.

2. Capsule selon la revendication précédente dans laquelle le composé aminé greffé en surface de celle-ci est par ailleurs lié de manière non covalente à une molécule d'intérêt.

3. Capsule selon l'une quelconque des revendications précédentes dans laquelle le composé aminé et/ou la molécule d'intérêt est de nature protéique, peptidique, nucléique, polymérique ou inorganique, voire organométallique.

4. Capsule selon l'une quelconque des revendications précédentes dans laquelle le composé aminé est une protéine choisie en particulier parmi l'albumine, la gélatine, les polypeptides, en particulier l'acide polyaspartique, la polyarginine, la polylysine ou un polymère synthétique, en particulier un polyéthylène imine.

5. Capsule selon l'une quelconque des revendications 1 à 4 dans laquelle le composé aminé et/ou la molécule d'intérêt est une molécule d'acide nucléique, en particulier un ARNsi.

**6.** Capsule selon l'une quelconque des revendications précédentes dont l'écorce lipidique comprend au moins un tensioactif liposoluble.

**7.** Capsule selon la revendication précédente dans laquelle le tensioactif liposoluble est choisi parmi les phospholipides, les lécithines et les phosphatidylcholines.

**8.** Capsule selon l'une quelconque des revendications précédentes, dont l'écorce lipidique solide est formée d'au moins un système tensioactif comprenant un tensioactif lipophile et, en outre, un tensioactif thermosensible, non ionique et hydrophile.

**9.** Capsule selon la revendication précédente dans laquelle le tensioactif thermosensible, non ionique et hydrophile est choisi parmi les phospholipides, les esters d'acide gras de sorbitol polyéthoxylé, les esters de polyéthylène glycol d'acide gras, des acides gras polyéthoxylés, des éthers d'alcool gras polyoxyéthylénés, des éthers non phényles polyoxyéthylénés, des éthers hydroxylphényle polyoxyéthylénés et un 2-hydroxystéarate polyéthylène glycol.

**10.** Capsule selon l'une quelconque des revendications précédentes, dont l'écorce lipidique comprend en outre au moins un ester d'alcool.

**11.** Capsule selon la revendication précédente, dans laquelle l'ester d'alcool est choisi parmi les esters d'alcool dont le nombre de carbone est un entier inférieur ou égal à 12, plus particulièrement inférieur ou égal à 10, encore plus particulièrement inférieur ou égal à 8.

**12.** Capsule selon l'une quelconque des revendications 10 et 11, dans laquelle l'ester d'alcool correspond à la formule suivante :

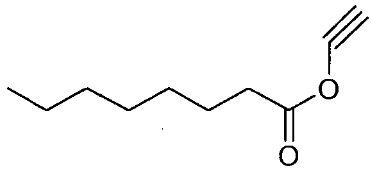

**13.** Capsule selon l'une quelconque des revendications précédentes, dans laquelle la réaction de transacylation est établie entre au moins un tensioactif liposoluble, présent dans l'écorce lipidique, et au moins un composé contenant au moins une fonction amine.

**14.** Capsule selon l'une quelconque des revendications précédentes, dans laquelle le composé aminé est greffé en surface de celle-ci via un procédé de transacylation à partir d'une lécithine, présente dans l'écorce lipidique.

**15.** Capsule selon l'une quelconque des revendications précédentes, dans laquelle la réaction de transacylation est établie entre au moins un ester d'alcool, présent dans l'écorce, et au moins un composé contenant au moins une fonction amine.

**16.** Capsule selon l'une quelconque des revendications précédentes, dans laquelle le coeur lipidique liquide comprend au moins une phase grasse huileuse formée d'au moins un corps gras liquide ou semi-liquide, en particulier d'au moins un triglycéride, d'un ester d'acide gras, ou d'un de leurs mélanges.

**17.** Capsule selon l'une quelconque des revendications précédentes dont l'architecture coeur/écorce lipidiques à l'échelle nanométrique encapsule au moins un actif dans le coeur et/ou l'écorce.

**18.** Procédé utile pour la fonctionnalisation de nanocapsules à écorce lipidique solide et coeur lipidique liquide, ledit procédé comprenant au moins les étapes consistant à :

i) disposer de nanocapsules à écorce lipidique solide et coeur lipidique liquide,
ii) mettre en contact lesdites nanocapsules avec une solution aqueuse alcaline, de manière à activer leur surface pour une réaction de transacylation,
iii) mettre en présence le milieu ii) avec au moins un composé contenant au moins une fonction amine, pour

former, par transacylation, lesdites capsules attendues et, le cas échéant,
iv) neutraliser les capsules fonctionnalisées ainsi obtenues, et
v) isoler lesdites capsules fonctionnalisées.

**19.** Procédé selon la revendication précédente dans lequel le composé aminé est un composé choisi parmi ceux mentionnés en revendications 3 à 5.

**20.** Procédé selon l'une des revendications 18 ou 19, dans lequel la réaction de transacylation met en oeuvre une alcalinisation par une solution de soude.

**21.** Capsules susceptibles d'être obtenues par le procédé selon l'une des revendications 18 à 20.

**22.** Utilisation d'au moins une capsule selon l'une quelconque des revendications 1 à 17 pour la préparation d'une composition à intérêt thérapeutique, cosmétique ou nutracetique.

**23.** Composition contenant au moins une capsule selon l'une quelconque des revendications 1 à 17 en association avec au moins un véhicule physiologiquement acceptable.

## Patentansprüche

**1.** Kapsel vom Typ flüssiger Lipidkern/feste Lipidschale, die an der Oberfläche mit mindestens einer Verbindung funktionalisiert ist, die mindestens eine Aminfunktion enthält, **dadurch gekennzeichnet, dass** Lipidkern/Lipidschale-Aufbau im Nanometermaßstab ist und dass die Verbindung über eine Transacylierungsreaktion kovalent an die feste Lipidschale gebunden ist.

**2.** Kapsel nach dem vorhergehenden Anspruch, wobei die auf die Oberfläche gepfropfte Aminoverbindung außerdem nicht-kovalent an ein Molekül von Interesse gebunden ist.

**3.** Kapsel nach einem der vorhergehenden Ansprüche, wobei die Aminoverbindung und/oder das Molekül von Interesse die Beschaffenheit eines Proteins, Peptids, einer Nukleinsäure, eines Polymers oder eines anorganisches Materials aufweist, insbesondere eines organometallischen Materials.

**4.** Kapsel nach einem der vorhergehenden Ansprüche, wobei die Aminoverbindung ein Protein ist, das insbesondere aus Albumin, Gelatine, Polypeptiden, insbesondere Polyasparaginsäure, Polyarginin, Polylysin, oder ein synthetisches Polymer ist, insbesondere ein Polyethylenimin.

**5.** Kapsel nach einem der Ansprüche 1 bis 4, wobei die Aminoverbindung und/oder das Molekül von Interesse ein Nukleinsäuremolekül ist, insbesondere siRNA.

**6.** Kapsel nach einem der vorhergehenden Ansprüche, wobei die Lipidschale mindestens ein lipidlösliches oberflächenaktives Mittel enthält.

**7.** Kapsel nach dem vorhergehenden Anspruch, wobei das lipidlösliche oberflächenaktive Mittel aus Phospholipiden, Lecithinen und Phosphatidylcholinen ausgewählt ist.

**8.** Kapsel nach einem der vorhergehenden Ansprüche, wobei die feste Lipidschale aus mindestens einem System eines oberflächenaktiven Mittels gebildet ist, umfassend ein lipophiles oberflächenaktives Mittel und weiterhin ein nicht-ionisches und hydrophiles wärmeempfindliches oberflächenaktives Mittel.

**9.** Kapsel nach dem vorhergehenden Anspruch, wobei das nicht-ionische und hydrophile wämeempfindliche oberflächenaktive Mittel aus Phospholipiden, Fettsäureestern von polyethoxyliertem Sorbitol, Polyethylenglycolestern von Fettsäuren, polyethoxylierten Fettsäuren, polyoxyethylenisierten Fettalkoholethern, polyoxyethylenisierten Nichtphenylethern, polyoxyethylenisierten Hydroxyphenylethern und 2-Hydroxystearat-Polyethylenglycol ausgewählt ist.

**10.** Kapsel nach einem der vorhergehenden Ansprüche, wobei die Lipidschale weiterhin mindestens einen Alkoholester enthält.

**11.** Kapsel nach dem vorhergehenden Anspruch, wobei der Alkoholester aus Alkoholestern ausgewählt ist, bei denen die Kohlenstoffzahl insgesamt kleiner oder gleich 12 ist, insbesondere kleiner oder gleich 10, weiterhin insbesondere kleiner oder gleich 8.

**12.** Kapsel nach Anspruch 10 oder 11, wobei der Alkoholester der folgenden Formel entspricht:

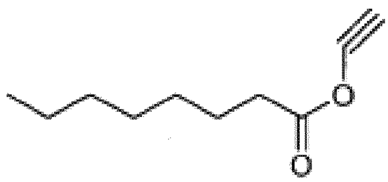

**13.** Kapsel nach einem der vorhergehenden Ansprüche, wobei die Transacylierungsreaktion zwischen mindestens einem lipidlöslichen oberflächenaktiven Mittel, das in der Lipidschale vorliegt, und mindestens einer Verbindung, die mindestens eine Aminfunktion enthält, bewerkstelligt wird.

**14.** Kapsel nach einem der vorhergehenden Ansprüche, wobei die Aminoverbindung über einen Transacylierungsvorgang unter Verwendung eines Lecithins, das in der Lipidschale vorliegt, auf die Oberfläche aufgepfropft ist.

**15.** Kapsel nach einem der vorhergehenden Ansprüche, wobei die Transacylierungsreaktion zwischen mindestens einem Alkoholester, der in der Schale vorliegt, und mindestens einer Verbindung, die mindestens eine Aminfunktion enthält, bewerkstelligt wird.

**16.** Kapsel nach einem der vorhergehenden Ansprüche, wobei der flüssige Lipidkern mindestens eine ölige Fettphase enthält, die aus mindestens einem flüssigen oder halbflüssigen Fettkörper gebildet ist, insbesondere aus mindestens einem Triglycerid, einem Fettsäureester oder einem Gemisch davon.

**17.** Kapsel nach einem der vorhergehenden Ansprüche, wobei der Lipidkern-/Lipidschale-Aufbau im Nanometermaßstab mindestens einen aktiven Wirkstoff im Kern und/oder in der Schale einkapselt.

**18.** Verfahren zur Funktionalisierung von Nanokapseln mit fester Lipidschale und flüssigem Lipidkern, wobei das Verfahren mindestens die folgenden Schritte aufweist:

i) Bereitstellen von Nanokapseln mit fester Lipidschale und flüssigem Lipidkern,
ii) Inkontaktbringen der Nanokapseln mit einer wässrigen Alkalilösung derart, dass deren Oberfläche für eine Transacylierungsreaktion aktiviert wird,
iii) Inkontaktbringen des Milieus ii) mit mindestens einer Verbindung, die mindestens eine Aminfunktion enthält, um mittels Transacylierung die Zielkapseln zu bilden, und, wenn nötig,
iv) Neutralisieren der so erhaltenen funktionalisierten Kapseln und
v) Isolieren der funktionalisierten Kapseln.

**19.** Verfahren nach dem vorhergehenden Anspruch, worin die Aminoverbindung eine Verbindung ist, die aus den in den Ansprüchen 3 bis 5 erwähnten Verbindungen ausgewählt ist.

**20.** Verfahren nach einem der Ansprüche 18 oder 19, worin die Transacylierungsreaktion eine Alkalisierung mittels einer Natriumhydroxidlösung bewerkstelligt.

**21.** Kapseln, die durch das Verfahren nach einem der Ansprüche 18 bis 20 erhältlich sind.

**22.** Verwendung mindestens einer Kapsel nach einem der Ansprüche 1 bis 17 zur Herstellung einer Zusammensetzung für therapeutische, kosmetische oder nutrazeutische Zwecke.

**23.** Zusammensetzung, welche mindestens eine Kapsel nach einem der Ansprüche 1 bis 17 zusammen mit mindestens einem physiologisch akzeptablen Vehikel enthält.

## Claims

1. A liquid lipid core/solid lipid shell capsule surface-functionalized with at least one compound containing at least one amine function, **characterized in that** the lipid core/lipid shell architecture is on the nanometric scale and **in that** said compound is covalently bonded to said solid lipid shell via a transacylation reaction.

2. The capsule as claimed in the preceding claim, in which the amino compound grafted at the surface of said capsule is, moreover, covalently bonded to a molecule of interest.

3. The capsule as claimed in either one of the preceding claims, in which the amino compound and/or the molecule of interest is of protein, peptide, nucleic, polymeric or inorganic nature, or even organometallic nature.

4. The capsule as claimed in any one of the preceding claims, in which the amino compound is a protein chosen in particular from albumin, gelatin, polypeptides, in particular poly(aspartic acid), polyarginine, polylysine or a synthetic polymer, in particular a polyethyleneimine.

5. The capsule as claimed in any one of claims 1 to 4, in which the amino compound and/or the molecule of interest is a nucleic acid molecule, in particular an siRNA.

6. The capsule as claimed in any one of the preceding claims, the lipid shell of which comprises at least one liposoluble surfactant.

7. The capsule as claimed in the preceding claim, in which the liposoluble surfactant is chosen from phospholipids, lecithins and phosphatidylcholines.

8. The capsule as claimed in any one of the preceding claims, the solid lipid shell of which is made up of at least one surfactant system comprising a lipophilic surfactant and also a nonionic hydro-philic thermosensitive surfactant.

9. The capsule as claimed in the preceding claim, in which the nonionic hydrophilic thermosensitive surfactant is chosen from phospholipids, poly-ethoxylated sorbitol fatty acid esters, esters of polyethylene glycol and of a fatty acid, polyethoxylated fatty acids, polyoxyethylenated fatty alcohol ethers, polyoxyethylenated nonphenyl ethers, polyoxyethylenated hydroxyphenyl ethers and a polyethylene glycol 2-hydroxystearate.

10. The capsule as claimed in any one of the preceding claims, the lipid shell of which also comprises at least one alcohol ester.

11. The capsule as claimed in the preceding claim, in which the alcohol ester is chosen from alcohol esters of which the carbon number is an integer less than or equal to 12, more particularly less than or equal to 10, even more particularly less than or equal to 8.

12. The capsule as claimed in either one of claims 10 and 11, in which the alcohol ester corresponds to the following formula:

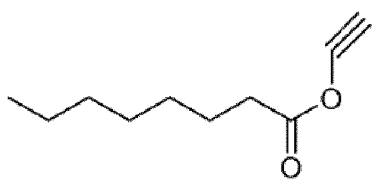

13. The capsule as claimed in any one of the preceding claims, in which the transacylation reaction is established between at least one liposoluble surfactant, present in the lipid shell, and at least one compound containing at least one amine function.

14. The capsule as claimed in any one of the preceding claims, in which the amino compound is grafted at the surface of said capsule via a transacylation method using a lecithin, present in the lipid shell.

15. The capsule as claimed in any one of the preceding claims, in which the transacylation reaction is established

between at least one alcohol ester, present in the shell, and at least one compound containing at least one amine function.

**16.** The capsule as claimed in any one of the preceding claims, in which the liquid lipid core comprises at least one oily fatty phase made up of at least one liquid or semi-liquid fatty substance, in particular at least one triglyceride, of a fatty acid ester, or of a mixture thereof.

**17.** The capsule as claimed in any one of the preceding claims, of which the lipid core/lipid shell architecture on the nanometric scale encapsulates at least one active agent in the core and/or the shell.

**18.** A method which is of use for the functionalization of nanocapsules comprising a solid lipid shell and a liquid lipid core, said method comprising at least the steps consisting in:

i) providing nanocapsules comprising a solid lipid shell and a liquid lipid core,
ii) bringing said nanocapsules into contact with an alkaline aqueous solution, so as to activate their surface for a transacylation reaction,
iii) bringing the medium ii) into contact with at least one compound containing at least one amine function, so as to form, by trans-acylation, said expected capsules and, where appropriate,
iv) neutralizing the resulting functionalized capsules, and
v) isolating said functionalized capsules.

**19.** The method as claimed in the preceding claim, in which the amino compound is a compound chosen from those mentioned in claims 3 to 5.

**20.** The method as claimed in either of claims 18 and 19, in which the transacylation reaction implements an alkalinisation via a sodium hydroxide solution.

**21.** Capsules which can be obtained by means of the method as claimed in one of claims 18 to 20.

**22.** The use of at least one capsule as claimed in any one of claims 1 to 17, for preparing a composition of therapeutic, cosmetic or nutraceutical interest.

**23.** A composition containing at least one capsule as claimed in any one of claims 1 to 17, in combination with at least one physiologically acceptable vehicle.

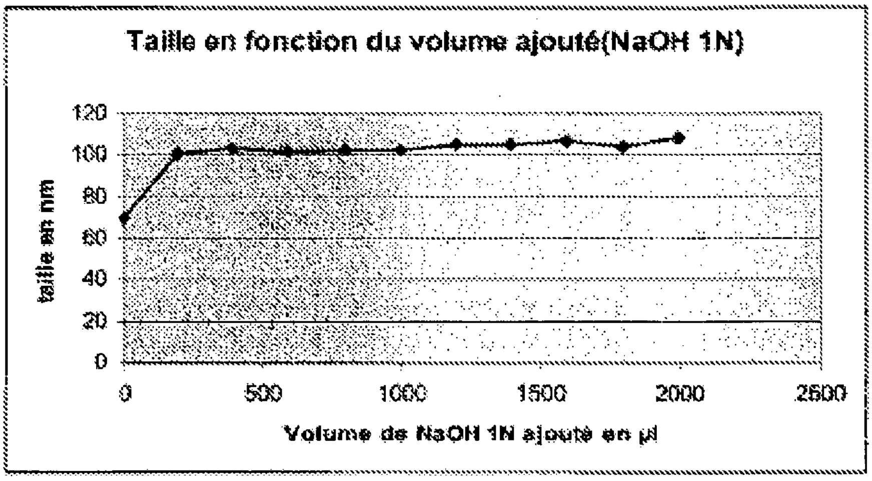


**FIGURE 1**

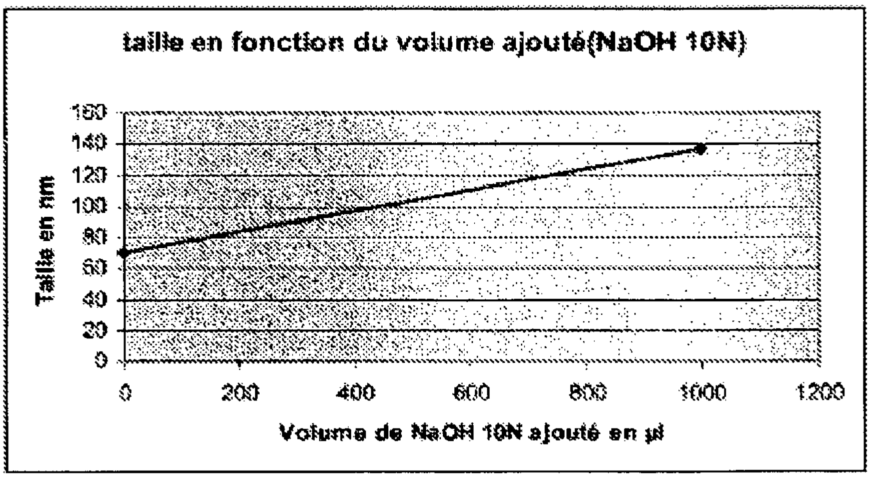


**FIGURE 2**

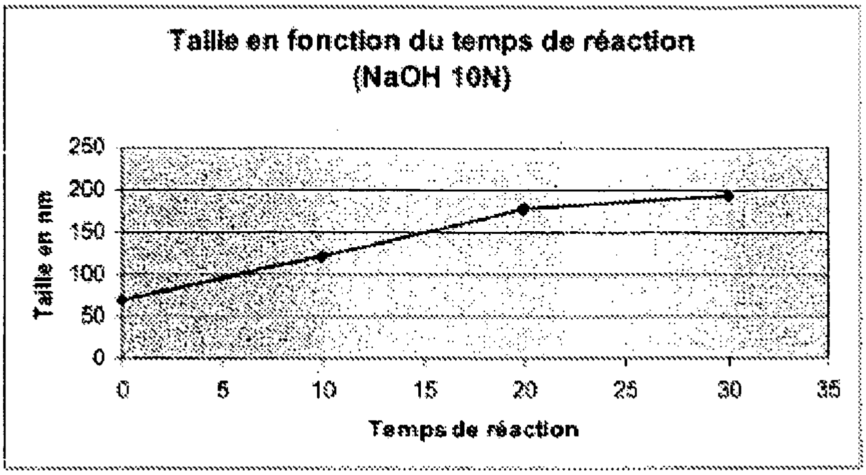


FIGURE 3

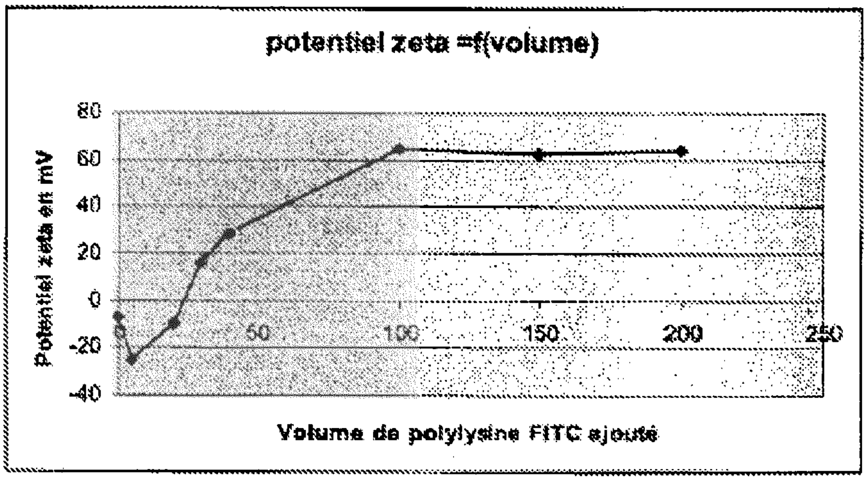


FIGURE 4

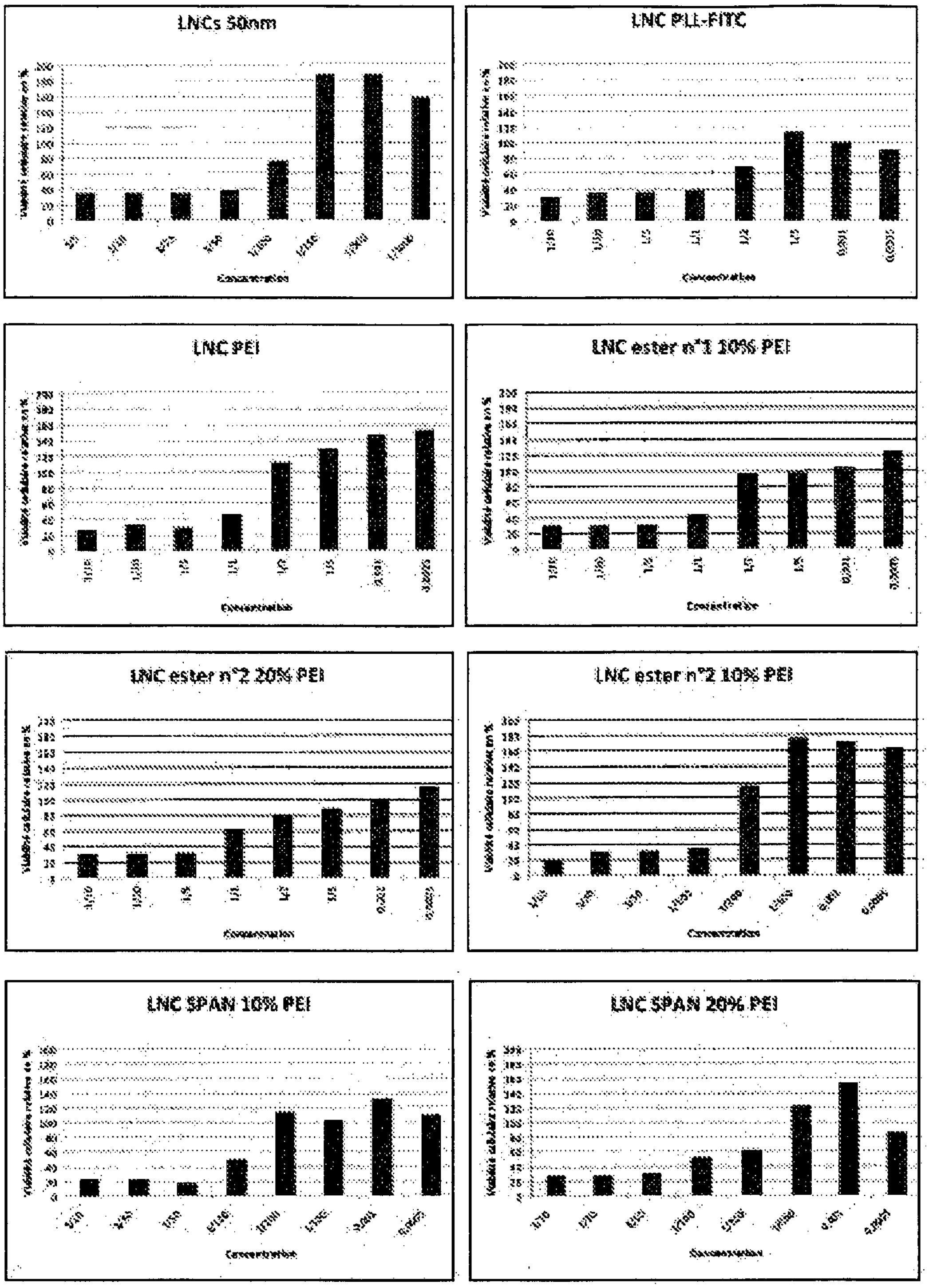
LNCs 50nm
LNC PLL-FITC
LNC PEI
LNC ester n°1 10% PEI
LNC ester n°2 20% PEI
LNC ester n°2 10% PEI
LNC SPAN 10% PEI
LNC SPAN 20% PEI

**FIGURE 5**

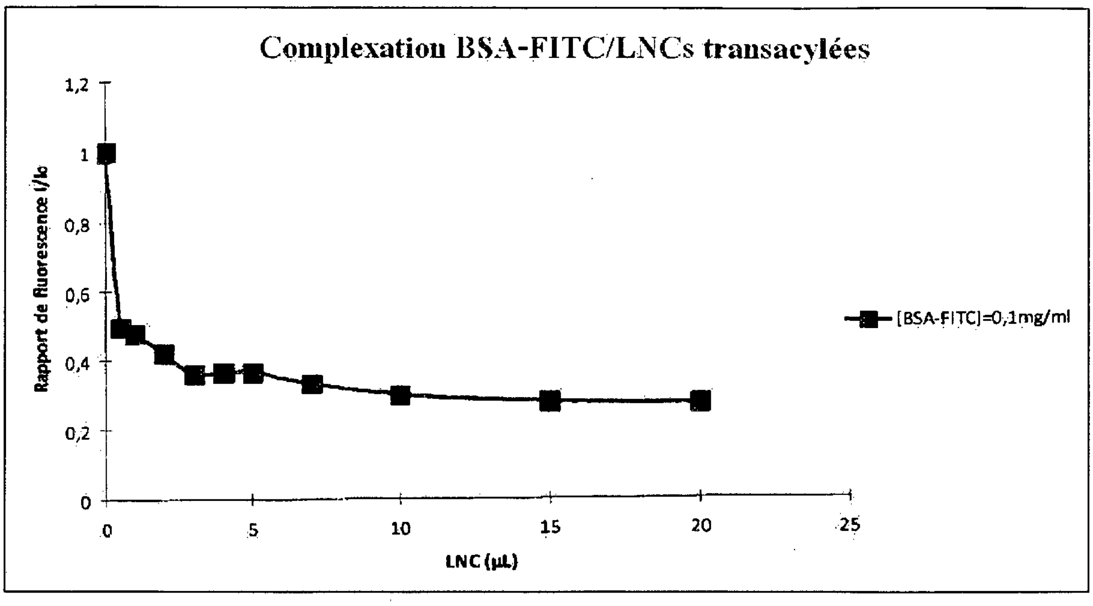


**FIGURE 6**

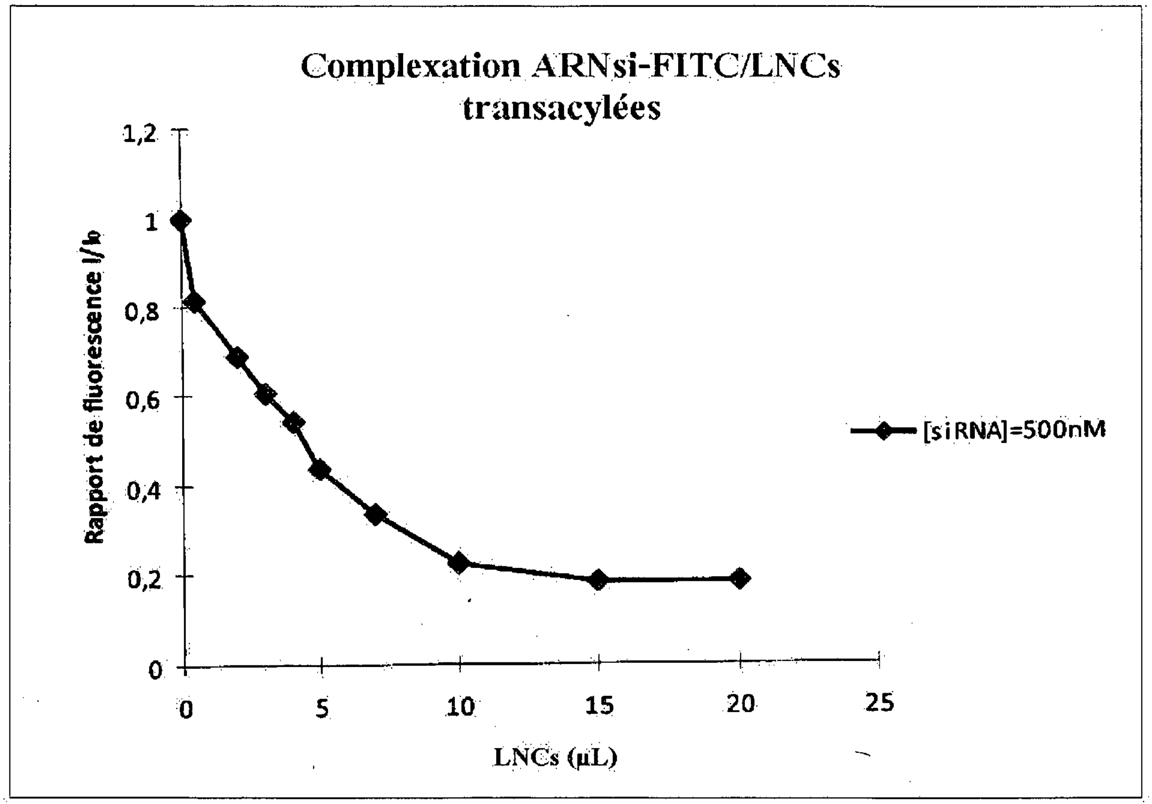


**FIGURE 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5961970 A
- US 5576016 A
- EP 1265698 A
- WO 09001019 A
- FR 2916974
- US 2008253960 A
- WO 2007106683 A
- FR 2677272
- EP 0693963 A


### Littérature non-brevet citée dans la description


- **MOSMANN T.** *Journal of immunological methods,* Décembre 1983, vol. 65 (1-2), 55-63
- **CORY et al.** *Cancer communications,* 1991, vol. 3 (7), 207-12